# EUROPÄISCHE PATENTANMELDUNG

(11) **EP 2 829 878 A1**
(43) Veröffentlichungstag der Anmeldung: **28.01.2015**
(21) Anmeldenummer: 13177569.4
(22) Anmeldetag: 23.07.2013
(51) Int. Cl.: G01N 33/543, G01N 33/86

(54) **Verfahren zur Bestimmung der GPIb-Rezeptor-abhängigen Plättchenfunktion**

(71) Anmelder: Siemens Healthcare Diagnostics Products GmbH, 35041 Marburg (DE)
(72) Erfinder: Patzke, Juergen, 35043 Marburg (DE)

(57) **Zusammenfassung**

Die vorliegende Erfindung liegt auf dem Gebiet der Plättchenfunktionsdiagnostik und betrifft ein Verfahren zur Bestimmung der GPIb-Rezeptor-abhängigen Plättchenfunktion, wobei ein Festphasen-gebundener anti-GPIb-Bindungspartner als Plättchenaktivator eingesetzt wird.

## Beschreibung

Die vorliegende Erfindung liegt auf dem Gebiet der Plättchenfunktionsdiagnostik und betrifft ein Verfahren zur Bestimmung der GPIb-Rezeptor-abhängigen Plättchenfunktion.

Physiologische Vorgänge, die einerseits die Fluidität des Blutes im Gefäßsystem gewährleisten und andererseits durch die Bildung von Blutgerinnseln die Vermeidung von extravasalen Blutverlusten sicherstellen, werden unter dem Begriff Hämostase zusammengefasst. An der Regulation der Hämostase sind eine Vielzahl von Proteinfaktoren beteiligt sowie auch zelluläre Komponenten, wie z.B. die Thrombozyten (Blutplättchen, Plättchen). Im Falle einer Gefäßverletzung kommt es zuerst zur Anlagerung von Thrombozyten an das subendotheliale Kollagen. Diese Adhäsion wird durch Adhäsivproteine, wie den von Willebrand Faktor (VWF) vermittelt. Während des Adhäsionsvorgangs werden die Plättchen aktiviert und schütten Mediatoren aus ihren Granulae aus, wodurch die Aggregation weiterer Plättchen sowie eine Verstärkung der Aktivierung induziert wird. Auf diese Weise erfolgt ein primärer Gefäßwandverschluss (primäre Hämostase), der erst durch weitere Reaktionen des plasmatischen Gerinnungssystems (sekundäre Hämostase) stabilisiert wird. Fehlregulationen dieser Prozesse können zu einer Thromboseneigung oder einer Blutungsneigung führen und je nach Schweregrad lebensbedrohliche Folgen haben.

In der Gerinnungsdiagnostik sind verschiedene Verfahren und Systeme bekannt, mit deren Hilfe bestimmt werden kann, ob das Blut eines Patienten einwandfrei gerinnen kann oder ob eine Gerinnungsstörung vorliegt. Im Fall einer Gerinnungsstörung ist es häufig erforderlich, präzisere Kenntnisse über die Ursache der vorliegenden Störung zu erlangen, um die optimalen therapeutischen Maßnahmen auswählen zu können. Eine wichtige Teilfunktion des Gerinnungssystems, die gezielt untersucht werden kann, ist die primäre Hämostase, die wesentlich von der Funktionsfähigkeit der Plättchen abhängt.

Im Stand der Technik sind verschiedene Verfahren zur Untersuchung der Plättchenfunktion in vitro bekannt.

Bei der Lichttransmissionsaggregometrie, die auch als Plättchenaggregation nach Born bezeichnet wird, wird die Aggregationsfähigkeit der Plättchen im plättchenreichen Plasma in Gegenwart von aggregationsinduzierenden Substanzen in einem Aggregometer photometrisch gemessen. Durch die Aggregatbildung wird die Lichtdurchlässigkeit der PRP-Probe erhöht, so dass durch die Messung der Lichtdurchlässigkeit z.B. die Geschwindigkeit der Aggregatbildung bestimmt werden kann. Mit Hilfe der Lichttransmissionsaggregometrie können auch therapeutische Effekte von medikamentös eingesetzten Thrombozytenaggregationshemmern erfasst werden.

Das VerifyNow® System (Accumetrics) ist eine Weiterentwicklung der Lichttransmissionsaggregometrie, die die Untersuchung der Plättchenfunktion in Vollblutproben ermöglicht. In diesem System wird die Aggregationsreaktion der Plättchen durch die Zugabe von Fibrinogen-beschichteten Mikropartikeln verstärkt.

Ein anderes Testprinzip zur Bestimmung der Plättchenfunktion ist das sogenannte Platelet Function Analyzer System, kurz PFA-System (PFA-100®, PFA-200, Siemens Healthcare Diagnostics Products GmbH, Marburg, Deutschland). Mit Hilfe des PFA-Systems wird in Vollblutproben die primäre Hämostase unter Flussbedingungen und damit in Gegenwart von hohen Scherkräften gemessen.

Ein anderes Testprinzip zur Bestimmung der Plättchenfunktion ist das Multiplate® System (Verum Diagnostica GmbH, München, Deutschland). Mit Hilfe des Multiplate-Systems wird in Vollblutproben die primäre Hämostase basierend auf der Impedanzaggregometrie gemessen. Dazu werden zwei Sensoreinheiten, die je aus zwei parallel angeordneten Sensordrähten bestehen und die in einer speziellen Messzelle angeordnet sind, in die Vollblutprobe eingetaucht. Durch die Zugabe von Plättchenaktivatoren, wie z.B. ADP, Kollagen oder Arachidonsäure, wird die Aggregation der Plättchen induziert. Die Plättchen heften sich an der Oberfläche der Sensordrähte an, wodurch sich der elektrische Widerstand zwischen den Sensordrähten erhöht. Die gemessene Erhöhung des elektrischen Widerstandes korreliert mit der Plättchenfunktion.

Wiederum andere Testprinzipien beruhen auf der Bestimmung von Granulae-Inhaltsstoffen, die infolge einer Aktivierung der Plättchen von den Plättchen sekretiert werden, wir z.B. ATP, P-Selektin, Plättchenfaktor 4 oder beta-Thromboglobulin.

Weitere Plättchenfunktionsteste sind beschrieben in Rand, M.L. et al., Platelet function assays. Transfusion and Apheresis Science 2003, 28: 307-317.

Allen Verfahren zur Untersuchung der Plättchenfunktion in vitro ist gemein, dass die zu untersuchenden Plättchen mit mindestens einem Plättchenaktivator in Kontakt gebracht werden. Bekannte Plättchenaktivatoren wie Kollagen, ADP, Thromboxan A₂, Arachidonsäure, Thrombin, Thrombin-Rezeptoraktivierende Peptide (TRAP-1, TRAP-4), Serotonin oder Epinephrin binden an spezifische Rezeptoren auf der Plättchenoberfläche, aktivieren verschiedene Signalwege und induzieren dadurch letztendlich die Aggregation der Plättchen. In Abhängigkeit von dem verwendeten Plättchenaktivator können also Defekte in dem aktivierten Signalweg detektiert werden.

Ein weiterer wichtiger Mechanismus der Plättchenfunktion ist die Interaktion zwischen dem Plättchenoberflächenrezeptor GPIb und dem plasmatischen VWF-Protein.

Der von Willebrand-Faktor (VWF) ist ein hochmolekulares, multimeres Glykoprotein im Blutplasma, das wichtige Funktionen in dem Prozess der primären Hämostase hat. Der VWF verfügt unter anderem über Bindungsstellen für Kollagen und für das Glykoprotein Ib (GPIb), das auf der Plättchenoberfläche lokalisiert ist. GPIb ist ein integrales Membranprotein und eine Komponente des GPIb-V-IX-Rezeptor-Komplexes. GPIb ist ein zweikettiges Molekül, das aus einer schweren Kette mit einer apparenten molekularen Masse von etwa 145 kDa (synonym: heavy chain, alpha-Kette oder GPIb**α**) und einer leichten Kette mit einer apparenten molekularen Masse von etwa 22 kDa (synonym: light chain, beta-Kette oder GPIb**β**) besteht, welche über Disulfidbrücken miteinander verbunden sind (Lopez, J.A. et al., Cloning of the α chain of human platelet glycoprotein Ib: A transmembrane protein with homology to leucine-rich α2-glycoprotein. Proc. Natl. Acad. Sci USA 1987, 84: 5615-5619). Glycocalicin ist ein Fragment der GPIb**α**-Kette, das proteolytisch von dem intakten Rezeptor in der Plättchenmembran abgespalten wird und im Plasma nachweisbar ist. Erhöhte Konzentrationen von freiem Glycocalicin im Plasma weisen auf eine Störung der Plättchenfunktion hin (Beer, J.H. et al., Glycocalicin: A New Assay - The Normal Plasma Levels And Its Potential Usefulness in Selected Diseases. Blood 1994, 83(3): 691-702).

Im Falle einer Gefäßverletzung werden Kollagenoberflächen exponiert, an die VWF bindet. Infolge der Bindung an Kollagen und unter dem Einfluss der erhöhten Scherkräfte, die auf den Kollagen-gebundenen VWF einwirken, wird der VWF so verändert oder aktiviert, dass er an das aminoterminale Ende der schweren Kette des GPIb (GPIb**α**) im GPIb-V-IX-Rezeptorkomplex der Thrombozytenmembran binden kann. Auf diese Weise fängt der aktivierte VWF vorbeiströmende Plättchen ein, so dass sich am Ort der Verletzung ein erstes Agglomerat aus VWF, Kollagen und Plättchen bildet. In der Folge werden die Plättchen aktiviert und damit auch die plasmatische Gerinnung in Gang gesetzt, die letztlich, nach mehreren Verstärkungskaskaden und der Anlagerung weiterer Plättchen, zum Wundverschluss führt. Störungen der VWF-GPIb-Interaktion führen zu einer erhöhten Blutungsneigung.

Die Methode zur Bestimmung der Funktionalität der VWF-GPIb-Interaktion ist die Plättchenagglutination mit Ristocetin. Bei diesem Verfahren wird eine Probe plättchenreichen Plasmas mit Ristocetin, einem nicht-physiologischen, antibiotischen Glykopeptid aus dem Bakterium Nocardia lurida, vermischt, und es wird die Plättchenaggregation bestimmt. Eine verminderte Plättchenaggregation indiziert, dass entweder eine qualitative oder quantitative VWF-Störung oder eine qualitative oder quantitative GPIb-Störung, wie z.B. das Bernard-Soulier-Syndrom, vorliegt. Zur genaueren Differenzierung der Störung bedarf es weiterer Teste, die spezifisch entweder eine VWF-Störung oder eine GPIb-Störung nachweisen.

Für den spezifischen Nachweis von quantitativen oder qualitativen Defekten des VWF ist eine Vielzahl von Testformaten bekannt. Für den spezifischen Nachweis von GPIb-Defekten existieren derzeit jedoch nur aufwändige und kaum standardisierte Methoden. Um den Einfluss des plasmatischen VWF aus der Patientenprobe auszuschließen, werden isolierte, gewaschene Plättchen als Probenmaterial eingesetzt. Die isolierten Plättchen werden dann mit Thrombin aktiviert oder mit exogenem VWF vermischt und mit Ristocetin aktiviert, und die Plättchenaggregation wird bestimmt. Nachteilig ist dabei, dass die Isolierung der Plättchen aus dem Blut oder Plasma aufwändig ist und außerdem das Risiko beinhaltet, dass es während der Isolierung zu einer vorzeitigen Aktivierung oder einer Schädigung der Plättchen kommt. Die Verwendung von Thrombin als Aktivator hat weiterhin den Nachteil, dass neben GPIb auch andere Rezeptoren auf der Plättchenoberfläche (PAR1, PAR4, GPV) aktiviert werden, so dass im Falle einer gestörten Thrombin-induzierten Plättchenaggregation nicht differenziert werden kann, welcher Rezeptor ursächlich für die Störung ist (De Candia, E., Mechanisms of platelet activation by thrombin: A short history. Thrombosis Research 2012, 129: 250-256).

Der vorliegenden Erfindung lag daher die Aufgabe zugrunde, ein Verfahren bereit zu stellen, das die Bestimmung der GPIb-Rezeptor-abhängigen Plättchenfunktion in Probenmaterial wie Blut oder Plasma ermöglicht, so dass auf die aufwändige Isolierung der Plättchen verzichtet werden kann.

Die Aufgabe wird erfindungsgemäß dadurch gelöst, dass die plättchenhaltige Probe mit einem Plättchenaktivator in Kontakt gebracht wird, der durch Bindung an den GPIb-Rezeptor die Plättchenaktivierung auslöst.

Es wurde gefunden, dass GPIb-Bindungspartner, insbesondere GPIb-bindende Antikörper, die an eine Festphase gebunden sind, als GPIb-spezifische Plättchenaktivatoren wirken. Überraschenderweise wurde außerdem festgestellt, dass inhibitorische GPIb-Antikörper, also GPIb-spezifische Antikörper, die in gelöster Form die Plättchenaktivierung durch VWF und Ristocetin inhibieren, dann, wenn sie an eine Festphase gebunden sind, genau entgegengesetzt, nämlich als Plättchenaktivatoren funktionieren.

Gegenstand der vorliegenden Erfindung ist also ein Verfahren zur Bestimmung der GPIb-Rezeptor-abhängigen Plättchenfunktion in einer plättchenhaltigen Probe, wobei die plättchenhaltige Probe mit einem Plättchenaktivator in Kontakt gebracht wird und die Plättchenaktivierung gemessen wird, und wobei der Plättchenaktivator ein Festphasen-gebundener, GPIb-Bindungspartner ist, und zwar vorzugsweise ein GPIb-spezifischer Bindungspartner.

Der Begriff "plättchenhaltige Probe" umfasst Flüssigkeiten, die Plättchen eines zu untersuchenden menschlichen oder tierischen Individuums enthalten, insbesondere biologische Flüssigkeiten, wie Blut oder Plasma. Auch wenn ein besonderer Vorteil des erfindungsgemäßen Verfahrens darin besteht, dass die Plättchen nicht aus dem natürlichen Probenmedium Blut oder Plasma isoliert werden müssen, so können dennoch auch isolierte Plättchen eines Individuums, die beispielsweise in einer Pufferlösung suspendiert sind, als Probe eingesetzt werden.

Der Plättchenaktivator, mit dem die plättchenhaltige Probe in dem erfindungsgemäßen Verfahren in Kontakt gebracht wird, ist ein Festphasen-gebundener, GPIb-Bindungspartner, vorzugsweise ein GPIb-spezifischer Bindungspartner.

Bevorzugte GPIb-Bindungspartner sind Antikörper, bevorzugt monoklonale Antikörper, die das GPIb-Protein binden. Bevorzugte GPIb-spezifische Antikörper binden an ein Epitop der GPIb**α**-Kette des GPIb-Proteins, vorzugsweise an ein Epitop der GPIb**α**-Kette, das von den Aminosäureresten 1-285 der GPIb**α**-Kette gebildet wird. Ganz besonders bevorzugt sind GPIb-spezifische Antikörper, die an ein Epitop der GPIb**α-**Kette binden, das von den Aminosäureresten 1-285 der GPIb**α-**Kette gebildet wird.

Die GPIb-Bindungspartner haben ferner bevorzugterweise die Eigenschaft, dass sie, wenn sie an GPIb gebunden sind, die zusätzliche Bindung von VWF an GPIb nicht komplett inhibieren.

Besonders bevorzugte GPIb-Bindungspartner sind monoklonale Antikörper, die an ein Epitop der GPIb**α**-Kette binden, das von den Aminosäureresten 1-285 der GPIb**α**-Kette gebildet wird, und die, wenn sie an die GPIb**α**-Kette gebunden sind, die zusätzliche Bindung von VWF an die GPIb**α**-Kette nicht oder zumindest nicht vollständig inhibieren. Derartige Antikörper sind aus dem Stand der Technik bekannt, wie z.B.
- der monoklonale Antikörper VM16d (Mazurov, A.V. et al., Characterization of an antiglycoprotein Ib monoclonals antibody that specifically inhibits platelet-thrombin interaction. Thromb Res. 1991, 62(6): 673-684; kommerziell erhältlich z.B. von Sanbio B.V., Uden, Niederlande:
   Produktnummer MON 1146);
- der monoklonale Antikörper SZ-2 (Ruan, C. et al., A murine antiglycoprotein Ib complex monoclonal antibody, SZ 2, inhibits platelet aggregation induced by both ristocetin and collagen. Blood 1987, 9(2): 570-577; kommerziell erhältlich z.B. von Beckman Coulter Inc., Fullerton, USA:
   Produktnummer IM0409); und
- der monoklonale Antikörper MB45 [siehe z.B. Modderman, P.W. et al., Glycoproteins V and Ib-IX form a noncovalent complex in the platelet membrane. J. Biol. Chem. 1992, 267(1): 364-369 und darin zitierte Referenz 26; kommerziell erhältlich z.B. von Sanquin, Amsterdam Niederlande:
   PeliCluster CD42b, Produktnummer M9142].

Weitere monoklonale Antikörper mit derartigen Eigenschaften sind beispielsweise erhältlich, indem in einem ersten Schritt durch Immunisierung eines Tieres, wie z.B. Maus, mit einem GPIb**α**-Peptid, das die Aminosäureresten 1-285 der GPIb**α**-Kette umfasst, und anschließender Etablierung von monoklonalen Hybridomazelllinien und Reinigung der sekretierten Antikörper zunächst GPIb-spezifische Antikörper hergestellt werden, die an ein Epitop der GPIb**α**-Kette binden, das von den Aminosäureresten 1-285 der GPIb**α**-Kette gebildet wird. In einem zweiten Schritt werden dann solche Antikörper selektiert, die, wenn sie an GPIb gebunden sind, die zusätzliche Bindung von VWF an GPIb nicht inhibieren. Dies kann beispielsweise erfolgen, indem die in Schritt 1 gewonnen Antikörper an eine Festphase gebunden werden und zunächst mit einer Lösung enthaltend ein GPIb**α**-Peptid, das die Aminosäureresten 1-285 der GPIb**α**-Kette umfasst inkubiert werden. Nach Entfernung des GPIb**α**-Peptid-haltigen Überstands und Waschen der Festphase wird dann die Festphase, die das Antikörper-gebundene GPIb**α**-Peptid aufweist, mit einer VWF-haltigen Lösung inkubiert. Nach Entfernung des VWF-haltigen Überstands und mehrmaligem Waschen der Festphase, kann dann geprüft werden, ob VWF-Protein an das Antikörper-gebundene GPIb**α**-Peptid gebunden wurde, beispielsweise mit Hilfe eines VWF-spezifischen Immunoassays (siehe auch Ausführungsbeispiel 3) .

Alternativ sind Antikörper mit den gewünschten Eigenschaften erhältlich, indem sie in dem zweiten Schritt des zuvor beschriebenen Herstellungsverfahrens dahingehend getestet werden, ob sie mit einem der bekannten Antikörper VM16d, SZ2 oder MB45 um die Bindung an das GPIb**α**-Peptid kompetieren. Dies kann beispielsweise erfolgen, indem die in Schritt 1 gewonnen Antikörper an eine Festphase gebunden werden und mit einer Lösung inkubiert werden, die einen Antigen-Antikörper-Komplex aus GPIb**α**-Peptid und einem der Antikörper VM16d, SZ2 oder MB45 enthält. Kompetiert ein Antikörper mit einem der Antikörper VM16d, SZ2 oder MB45, d.h. ist eine Bindung des Antigen-Antikörper-Komplex aus GPIb**α**-Peptid und einem der Antikörper VM16d, SZ2 oder MB45 an den neu hergestellten Antikörper nicht nachweisbar, ist er für die Verwendung in dem erfindungsgemäßen Verfahren geeignet.

Unter dem Begriff "Antikörper" ist im Sinne dieser Erfindung ein Immunglobulin, z.B. ein Immunglobulin der Klasse bzw. Subklasse IgA, IgD, IgE, IgG₁, IgG₂ₐ, IgG_{2b}, IgG₃, IgG₄, IgM, zu verstehen. Unter dem Begriff Antikörper sind jedoch nicht nur komplette Antikörper zu verstehen, sondern ausdrücklich auch Antikörperfragmente, wie z.B. Fab, Fv, F(ab')₂, Fab', sofern die Bindungseigenschaften an das GPIb-Protein erhalten sind. Antikörperfragmente lassen sich beispielsweise durch enzymatische Spaltung von Antikörpern mit Enzymen wie Pepsin oder Papain herstellen.

Ein GPIb-Bindungspartner ist an eine Festphase gebunden.

Der Begriff "Festphase" im Sinne dieser Erfindung beinhaltet einen Gegenstand, der aus porösem und/oder nicht porösem, wasserunlöslichem Material besteht und der die unterschiedlichsten Formen aufweisen kann, wie z.B. Gefäß, Röhrchen, Mikrotitrationsplatte (ELISA-Platte), Kugel, Mikropartikel, Stäbchen, Streifen, Filter- oder Chromatographiepapier, etc. In der Regel ist die Oberfläche der Festphase hydrophil oder kann hydrophil gemacht werden. Die Festphase kann aus den unterschiedlichsten Materialien bestehen wie z.B. aus anorganischen und/oder aus organischen Materialien, aus synthetischen, aus natürlich vorkommenden und/oder aus modifizierten natürlich vorkommenden Materialien. Beispiele für Festphasenmaterialien sind Polymere wie z.B. Cellulose, Nitrocellulose, Zelluloseacetat, Polyvinylchlorid, Polyacrylamid, vernetzte Dextranmoleküle, Agarose, Polystyrol, Polyethylen, Polypropylen, Polymethacrylat oder Nylon; Latex; Keramik; Glas; Metalle, insbesondere Edelmetalle wie Gold und Silber; Magnetit; Mischungen oder Kombinationen derselben.

Die Festphase kann einen Überzug aus einer oder mehreren Schichten aufweisen, z.B. aus Proteinen, Kohlehydraten, lipophilen Substanzen, Biopolymeren, organischen Polymeren oder Mischungen hiervon, um beispielsweise die unspezifische Bindung von Probenbestandteilen an die Festphase zu unterdrücken oder zu verhindern, oder um beispielsweise Verbesserungen zu erreichen hinsichtlich der Suspensionsstabilität von partikulären Festphasen, der Lagerstabilität, der formgebenden Stabilität oder der Resistenz gegen UV-Licht, Mikroben oder sonstige zerstörend wirkende Agenzien.

In einer bevorzugten Ausführungsform des erfindungsgemäßen Verfahrens ist der GPIb-Bindungspartner an eine partikuläre Festphase, bevorzugterweise an Latexpartikel gebunden.

Unter dem Begriff "partikuläre Festphase" sind im Sinne dieser Erfindung Teilchen zu verstehen, die einen ungefähren Durchmesser von wenigstens 20 nm und nicht mehr als 20 **µ**m aufweisen, üblicherweise zwischen 200 nm und350 nm, bevorzugt zwischen 250 und 320 nm, besonders bevorzugt zwischen 270 und 290 nm, ganz besondersbevorzugt 280 nm. Die Mikropartikel können regelmäßig oder unregelmäßig geformt sein. Sie können Kugeln, Spheroide, Kugeln mit mehr oder weniger großen Kavitäten oder Poren darstellen. Die Mikropartikel können aus organischem, aus anorganischem Material oder aus einer Mischung oder einer Kombination von beiden bestehen. Sie können aus einem porösen oder nicht porösen, einem schwellfähigen oder nicht schwellfähigen Material bestehen. Prinzipiell können die Mikropartikel jegliche Dichte haben, bevorzugt sind jedoch Partikel mit einer Dichte, die der Dichte des Wassers nahe kommt wie etwa 0,7 bis etwa 1,5 g/ml. Die bevorzugten Mikropartikel sind in wässrigen Lösungen suspendierbar und möglichst lange suspensionsstabil. Sie mögen durchsichtig, teilweise durchsichtig oder undurchsichtig sein. Die Mikropartikel können aus mehreren Schichten bestehen wie beispielsweise die sogenannten "coreand-shell" Partikel mit einem Kern und einer oder mehreren umhüllenden Schichten. Der Begriff Mikropartikel umfasst beispielsweise Farbstoffkristalle, Metallsolen, Silica-Partikel, Glaspartikel und Magnetpartikel. Bevorzugte Mikropartikel sind in wässrigen Lösungen suspendierbare und aus wasserunlöslichem Polymermaterial bestehende Partikel, insbesondere aus substituierten Polyethylenen. Ganz besonders bevorzugt sind Latexpartikel z.B. aus Polystyrol, Acrylsäurepolymeren, Methacrylsäurepolymeren, Acrylnitril-Polymeren, Acrylnitril-Butadien-Styrol, Polyvinylacetat-Acrylat, Polyvinylpyridin, Vinylchlorid-Acrylat. Von besonderem Interesse sind Latexpartikel mit reaktiven Gruppen an ihrer Oberfläche wie beispielsweise Carboxyl-, Amino- oder Aldehydgruppen, die eine kovalente Bindung des GPIb-spezifischen Bindungspartners an die Latexpartikel erlauben. Die Herstellung von Latexpartikeln ist beispielsweise in EP-A2-0080614, EP-A2-0227054 und EP-A2-0246446 beschrieben.

Der Begriff "gebunden" ist breit zu verstehen und umfasst beispielsweise eine kovalente und eine nicht-kovalente Bindung, eine direkte und eine indirekte Bindung, die Adsorption an eine Oberfläche und den Einschluss in eine Vertiefung. Bei einer kovalenten Bindung ist der GPIb-spezifische Bindungspartner über eine chemische Bindung an die Festphase gebunden. Ein Beispiel für eine nicht-kovalente Bindung ist die Oberflächenadsorption. Neben einer direkten Bindung an die Festphase kann der GPIb-spezifische Bindungspartner auch indirekt über spezifische Wechselwirkung mit anderen spezifischen Bindungspartnern an die Festphase gebunden sein, z.B. über spezifische Wechselwirkung mit einem Antikörper. Handelt es sich bei dem GPIb-spezifischen Bindungspartner beispielsweise um einen monoklonalen Antikörper aus der Maus kann die Bindung des GPIb-spezifischen Bindungspartners über einen direkt mit der Festphase verbundenen anti-Maus-Antikörper erfolgen.

Zur Messung der Plättchenfunktion in dem erfindungsgemäßen Verfahren eignet sich jedes Verfahren, das die Aktivierung der Plättchen infolge des In-Kontakt-Bringens mit dem erfindungsgemäßen Plättchenaktivator quantitativ zu erfassen vermag. Geeignete Verfahren zur Messung der Plättchenfunktion sind beispielsweise die Messung der Plättchenaggregation im Reaktionsansatz durch Messung der Änderung der Lichttransmissivität des Reaktionsansatzes (Lichttransmissionsaggregometrie) oder, sofern Vollblut als Probenmaterial verwendet wird, Messung der Plättchenaggregation im Reaktionsansatz durch Impedanzaggregometrie. Ein anderes geeignetes Testprinzip zur Bestimmung der Plättchenfunktion ist das sogenannte Platelet Function Analyzer System, bei dem in Vollblutproben unter Verwendung spezieller Messzellen die Zeit für den Verschluss einer Apertur unter Flussbedingungen und damit in Gegenwart von hohen Scherkräften gemessen wird. Weitere Verfahren zur Messung der Plattchenaktivität umfassen die Bestimmung von sekretierten Granulae-Inhaltsstoffen oder der Expression von Aktivierungmarkern auf der Plättchenoberfläche mittels Immunoassays oder Durchflusszytometrie.

In einer bevorzugten Ausführungsform des erfindungsgemäßen Verfahrens wird die Plättchenaktivierung gemessen, indem die Plättchenaggregation in der Probe gemessen wird, vorzugsweise mittels Lichttransmissionsaggregometrie.

Das erfindungsgemäße Verfahren zur Bestimmung der GPIb-Rezeptor-abhängigen Plättchenfunktion eignet sich insbesondere zur Diagnose eines Bernard-Soulier-Syndroms, einer hereditären Thrombozytopathie, der eine Störung der Plättchenadhäsion zu Grunde liegt, die wiederum auf einen erblich bedingten GPIb-Defekt zurückgeht. Eine mit dem erfindungsgemäßen Verfahren gemessene und gegenüber dem Normal verminderte oder fehlende GPIb-Rezeptor-abhängige Plättchenfunktion indiziert ein Bernard-Soulier-Syndrom.

Weiterhin eignet sich das erfindungsgemäße Verfahren zur Bestimmung der GPIb-Rezeptor-abhängigen Plättchenfunktion zur qualitativen oder quantitativen Bestimmung eines GPIb-Inhibitors in einer Probe. GPIb-Inhibitoren sind z.B. Autoantikörper gegen GPIb, die die Bindung des VWF an GPIb verhindern oder Therapeutika, wie z.B. H6B4-Fab, das Fab-Fragment eines humanisierten monoklonalen anti-GPIb**α-**Antikörpers (siehe Firbas, C. et al., Targeting von Willebrand factor and platelet glycoprotein Ib receptor. Expert Rev. Cardiovasc. Ther. 2010, 8(12): 1689-1701). Eine mit dem erfindungsgemäßen Verfahren gemessene und gegenüber dem Normal verminderte oder fehlende GPIb-Rezeptor-abhängige Plättchenfunktion indiziert die Anwesenheit eines GPIb-Inhibitors in der Probe.

Darüberhinaus könnte mit dieser Methode die Funktionalität eines möglicherweise bisher unbekannten GPIb-vermittelten Aktivierungsweges der Plättchen erfasst werden. Dieser Aktivierungsweg ist nicht abhängig von Thrombin oder VWF.

Ein weiterer Gegenstand der vorliegenden Erfindung ist die Verwendung eines GPIb-Bindungspartners, vorzugsweise eines spezifischen GPIb- Bindungspartners, der an eine Festphase gebunden ist, zur Aktivierung von Plättchen in vitro. GPIb-Bindungspartner, die an eine Festphase gebunden sind, sind bekannt. Die WO-A2-2009007051 beschreibt insbesondere GPIb-spezifische Antikörper, die an Latexpartikel gebunden sind. Die Latexpartikel werden dabei für den quantitativen Nachweis von plasmatischem VWF in einer Plasmaprobe verwendet. Dass sich GPIb-spezifische Bindungspartner, die an eine Festphasen gebunden sind, für die Verwendung als Plättchenaktivatoren eignen, also in der Lage sind, Plättchen über die Bindung an den GPIb-Rezeptor zu aktivieren und damit letztendlich eine Aggregationsreaktion zu induzieren, wird erstmals mit der vorliegenden Erfindung bekannt.

Besonders bevorzugt ist die Verwendung eines Festphasen-gebundenen GPIb-Antikörpers oder eines Festphasen-gebundenen Antikörperfragments mit Spezifität für GPIb zur Aktivierung von Plättchen in vitro.

Bevorzugterweise ist der GPIb-spezifischen Bindungspartner an eine partikuläre Festphase, vorzugsweise an Latexpartikel, gebunden.

Die Verwendung eines GPIb-spezifischen Bindungspartners, der an eine Festphase gebunden ist, zur Aktivierung von Plättchen in vitro eignet sich inbesondere zur Durchführung von in vitro-diagnostischen Verfahren.

### FIGURENBESCHREIBUNG

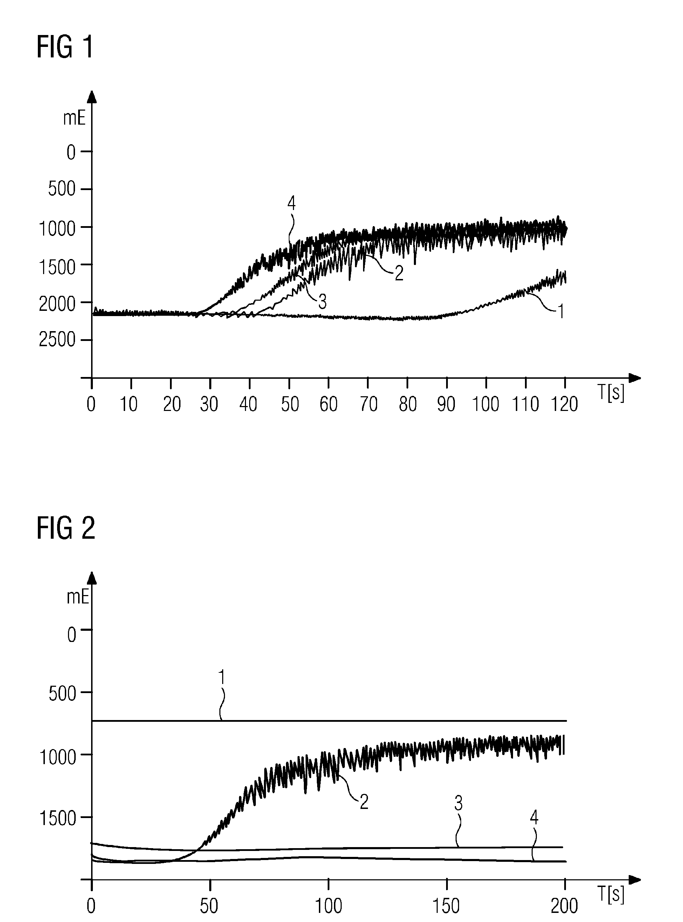

- FIG. 1: zeigt die Plättchenaggregationskurven, die nach Zugabe verschiedener erfindungsgemäßer Plättchenaktivatoren (Latexpartikel-gekoppelte anti-GPIb-Antikörper) zu plättchenreichem Plasma gemessen werden (Beispiel 1); Kurve 1: VM16d, Kurve 2: 2007-101/044; Kurve 3: 2007-98/069, Kurve 4: 2007-100/05.
- FIG. 2: zeigt die Plättchenaggregationskurven, die nach Zugabe von Latexpartikel-gekoppeltem anti-GPIb-Antikörper 2007-100/05 als Plättchenaktivator und von CTAD und PGE1 als Plättcheninhibitoren zu plättchenreichem Plasma gemessen werden (Beispiel 1). Kurve 1: plättchenarmes Plasma als Kontrolle ohne Zugabe von Plättchenaktivator oder Plättcheninhibitor, Kurve 2: plättchenreiches Plasma mit Plättchenaktivator, aber ohne Plättcheninhibitor, Kurve 3: plättchenreiches Plasma mit Plättchenaktivator und mit CTAD, Kurve 4: plättchenreiches Plasma mit Plättchenaktivator und mit PGE1.

### BEISPIELE

### Beispiel 1: Verwendung von an Latexpartikel gebundenen GPIb-spezifischen Antikörpern als Plättchenaktivatoren

Zur Herstellung von Latexpartikel-Reagenzien wurden die monoklonalen anti-GPIbα-Antikörper VM16d (Sanbio B.V., Uden, Niederlande: Produktnummer MON 1146), 2007-100/05, 2007-98/069 und 2007-101/044 (Letztere siehe Beispiel 3) jeweils auf der Oberfläche von Latexpartikeln (Durchmesser ca. 280 nm) gebunden, und die Partikel wurden dann in einem Puffer zur Langzeitlagerung resuspendiert (0,6 g/L TRIS, 1,1 % Leucin, 12,5-12,8 % Saccharose, 0,05 % HSA, 6,25 mg/L Gentamycin und 0,625 mg/L Amphotericin, pH 8.25, Latexkonzentration 0,22 %).

135 **µ**L plättchenreichen Plasmas eines gesunden Spenders wurden mit jeweils 15 **µ**L der vorgenannten Latexpartikel-Reagenzien (jeweils 2 g Latex /L Latexreagenz) zu einem Reaktionsansatz vermischt, und die Plättchenaggregation im Reaktionsansatz wurde durch Messung der Lichtextinktion über die Zeit bestimmt (siehe FIG. 1; Kurve 1: VM16d, Kurve 2: 2007-101/044; Kurve 3: 2007-98/069, Kurve 4: 2007-100/05).

Jeder der vier an Latexpartikel gebundenen anti-GPIbα-Antikörper löst eine Plättchenaggregation im Reaktionsansatz aus und hat damit eindeutig eine Plättchen-aktivierende Wirkung. Zwar ist die Lag-Phase der Plättchenaggregationsreaktion in den verschiedenen Ansätzen unterschiedlich lang, aber ist die Aktivierung der Plättchen einmal ausgelöst, erfolgt sie unabhängig vom eingesetzten Antikörper bis zur gleichen Maximalaggregation, welches durch das gleiche Endniveau der Extinktion gekennzeichnet ist.

Im Gegensatz dazu löst die Zugabe derselben Antikörper in ungebundenem Zustand, d.h. nicht an eine Festphase gebunden, keine Plättchenaggregation in plättchenreichem Plasma aus (siehe Tabelle 1).

**Tabelle 1: Messung der Plättchenaggregation (mE/sec) nach Zugabe verschiedener nicht an eine Festphase gebundener Antikörper zu plättchenreichem Plasma**

| | anti-GPIbα-Antikörper | | | |
|---|---|---|---|---|
| Konzentration des ungebundenen Antikörpers (µg/mL) | 2007-100/05 | 2007-98/069 | 2007-101/044 | VM1 6d |
| 0 | 0,5 | 0, 7 | 0, 4 | 0, 4 |
| 2 | 0,5 | 0, 4 | 0, 6 | 0, 6 |
| 5 | 0,5 | 0, 4 | 0, 4 | 0,5 |
| 10 | 0,3 | 0,5 | 0, 6 | 0, 6 |
| 20 | 0,5 | 0, 6 | 0, 4 | 0, 4 |
| 50 | 0,5 | 0,3 | 0, 4 | 0, 6 |
| | | | | |
| Latexpartikelgebundener Antikörper (2g /L) | 19, 9 | 18,1 | 13, 6 | 6,3 |

Ferner wurde beobachtet, dass die Zugabe eines anti-GPIbα-Antikörpers in ungebundenem Zustand die plättchenaktivierende Wirkung desselben anti-GPIbα-Antikörpers, der an eine Festphase gebunden ist, hemmt. Dazu wurde eine Probe plättchenreichen Plasmas vor Zugabe eines erfindungsgemäßen Plättchenaktivators bestehend aus Antikörper-beschichteten Latexpartikeln mit einem Antikörper in ungebundenem Zustand präinkubiert, und die Plättchenaggregation wurde gemessen (mE/sec). Wie aus Tabelle 2 hervorgeht, hemmen die ungebundenen Antikörper SZ2, 2007-100/05 und VM16d die plättchenaktivierende Wirkung eines erfindungsgemäßen Plättchenaktivators bestehend aus dem anti-GPIbα-Antikörper 2007-100/05, der an Latexpartikel gebunden ist.

**Tabelle 2: Messung der Plättchenaggregation (mE/sec) in verschiedenen Reaktionsansätzen**

| Zugabe von ungebundenem anti-GPIbα-Antikörper | Zugabe von an Latexpartikel gebundenem anti-GPIbα-Antikörper 2007-100/05 als Plättchenaktivator | Zugabe von Ristocetin als Plättchenaktivator (Ristocetin-Kofaktor-Test) |
|---|---|---|
| Negativkontrolle | 16 | 25 |
| AK2 | 16 | 1 |
| SZ2 | 9 | 21 |
| 2007-100/05 | 1 | 26 |
| VM1 6d | 1 | 29 |

Vom Antikörper AK2 ist bekannt, dass er an die Bindungsstelle des VWF auf dem GPIb-Rezeptor bindet und daher den Ristocetin-Kofaktor-Test hemmt (Ward, C.M. et al., Mocarhagin, a novel cobra venom metalloproteinase, cleaves the platelet von Willebrand factor receptor glycoprotein Ibα. Identification of the sulfated tyrosine/anionic sequence Tyr-276-Glu-282 of glycoprotein Ibα as a binding site for von Willebrand factor and α-thrombin. Biochemistry 1996, 35: 4929-4938). Daher wird die Aktivierung der Plättchen durch den an Latexpartikel gebundenen anti-GPIbα-Antikörper 2007-100/05, der -wenn er an GPIb gebunden istdie zusätzliche Bindung von VWF an GPIb nicht inhibiert, also nicht an die Bindungsstelle des VWF auf dem GPIb-Rezeptor bindet, nicht durch den Antikörper AK2 gehemmt. Der Antikörper SZ2 hemmt die Plättchenaggregation im Ristocetin-Kofaktor-Test bekanntermaßen teilweise (Shen, Y. et al., Requirement of leucine-rich repeats of glycoprotein (GP) Ibα for shear-dependent and static binding of von Willebrand factor to the platelet membrane GPIb-IX-V complex. Blood 2000, 95(3): 903-910). Dieser Antikörper hemmt hier ebenfalls teilweise die Aktivierung der Plättchen mit einem erfindungsgemäßen Plättchenaktivator. Die Zugabe der Antikörper 2007-100/05 oder VM16d hemmt den Ristocetin-Kofaktor-Test nicht, aber die Aktivierung der Plättchen mit einem erfindungsgemäßen Plättchenaktivator, weil die ungebundenen Antikörper offensichtlich die Bindungsstellen des Plättchenaktivators bestehend aus an Latexpartikeln gebundenen Antikörpern absättigen, so dass der erfindungsgemäße Plättchenaktivator nicht mehr binden kann.

In FIG. 2 wird gezeigt, dass die erfindungsgemäße Plättchenaktivierung durch Festphasen-gebundene anti-GPIb**α-**Antikörper in Anwesenheit von Inhibitoren der Plättchenaktivierung gehemmt wird. Dazu wurden Reaktionsansätzen, in denen die Plättchenaggregation mit dem Latex-gebundenen anti-GPIbα-Antikörper 2007-100/05 induziert wurde, der Plättchenaktivierungs-Inhibitor Prostaglandin E1 (PGE1) oder CTAD (Citrat, Theophyllin, Adenosin, Dipyridamol) zugegeben.

### Beispiel 2: Identifizierung von anti-GPIbα-Antikörpern, die -wenn sie an GPIb gebunden sind- die zusätzliche Bindung von VWF an GPIb nicht inhibieren

Es wurden die folgenden kommerziell verfügbaren monoklonalen Maus-Antikörper getestet, die bekanntermaßen spezifisch humanes GPIb**α**-Protein binden: VM16d, Alma19, SZ2, MB45, MB15, MM2/174, AK2, AB-1 und HIP1.

Die Vertiefungen einer ELISA-Platte wurden mit polyklonalen anti-Maus-IgG Antikörpern beschichtet. Anschließend wurden jeweils 200 µL einer Antikörperlösung mit einem der vorgenannten Antikörper (1 µg/mL) in jeweils eine Vertiefung gegeben. Nach 1 h Inkubation bei Raumtemperatur wurde der Überstand entfernt, und die Vertiefungen wurden viermal mit 300 µL Phosphatpuffer gewaschen. In jede Vertiefung wurden dann 100 µL einer GPIb**α**-Lösung (rekombinant hergestelltes GPIb**α**-Fragment (aa 1-285, 2,5 µg/mL) bzw. als Kontrolle Phosphatpuffer ohne GPIb**α** gegeben. Nach 1 h Inkubation bei Raumtemperatur wurde der Überstand entfernt, und die Vertiefungen wurden viermal mit 300 µL Phosphatpuffer gewaschen. In jede Vertiefung wurden dann 50 uL Standard Human Plasma und 50 µL Phophatpuffer mit 2 mg/mL Ristocetin gegeben. Nach 1 h Inkubation zur Bindung des plasmatischen VWF an das Festphasen-gebundene GPIb**α** wurde der Überstand entfernt, und die Vertiefungen wurden viermal mit 300 µL Phosphatpuffer gewaschen. In jede Vertiefung wurden dann 100 µL eines Meerrettichperoxidase-gekoppelten anti-human VWF-Antikörpers (1:220 = 5 µg/mL) gegeben. Nach Inkubation und anschließendem Waschen wurde eine TMB-Substratlösung in jede Vertiefung gegeben und schließlich die Extinktion bei 450 nm gemessen. Um mögliche unspezifische Bindungen des VWF zu berücksichtigen, wurde aus den Messungen der Ansätze mit GPIb**α**-Fragment und einem Kontrollansatz, dem kein GPIb**α-**Fragment zugegeben wurde, die Differenz gebildet. Je höher der gemessene Wert bzw. die berechnete Differenz desto mehr VWF wurde von dem Antikörper-gebundenen GPIb**α**-Fragment gebunden. Die Ergebnisse sind in Tabelle 2 dargestellt.

**Tabelle 2: Quantitative Bestimmung der Bindung von VWF an Antikörper-gebundenes GPIbα-Fragment**

| | | | | | | | | | |
|---|---|---|---|---|---|---|---|---|---|
| Ristocetin (2 mg/mL) | **VM16d** | ALMA 19 | **SZ2** | **MB45** | MB15 | MM2/ 174 | AK2 | AB-1 | HIP1 |
| Mit GPIb | **3573** | 168 | **3742** | **4092** | 276 | 273 | 204 | 240, | 154 |
| Ohne GPIb | **1141** | 211 | **415** | **521** | 502 | 250 | 208 | 0 191 | 201 |
| Differenz | **2432** | -43 | **3327** | **3571** | -226 | 23 | -4 | 49 | -47 |
| | | | | | | | | | |
| Ohne Ristocetin | **VM16d** | ALMA 19 | **SZ2** | **MB45** | MB15 | MM2/ 174 | AK2 | AB-1 | HIP1 |
| Mit GPIb | **4087** | 93 | **1259** | **3993** | 232 | 259 | 253 | 193, | 154 |
| Ohne GPIb | **105** | 95 | **285** | **268** | 221 | 245 | 233 | 0 192 | 152 |
| Differenz | **3982** | -2 | **974** | **3725** | 11 | 14 | 20 | 1 | 2 |

Die Antikörper VM16d, SZ2 und MB45 binden das GPIb**α**-Fragment räumlich derart, dass die VWF-Bindung an das GPIb**α**-Fragment nicht gestört ist.

### Beispiel 3: Herstellung von anti-GPIbα-Antikörpern, die -wenn sie an GPIb gebunden sind- die zusätzliche Bindung von VWF an GPIb nicht inhibieren

Es wurden 18 Mäuse mit einem rekombinant hergestellten GPIb**α**-Peptid (aa 1-285) immunisiert. Immunisierung und Fusion der Milzzellen mit Myelomzellen erfolgen nach den dem Fachmann bekannten Methoden zur Herstellung von monoklonalen Antikörpern.

In einem ersten Screening-Schritt wurden 814 Hybridomazelllinien im Hinblick auf die Synthese von Antikörpern mit Spezifität für das GPIb**α**-Peptid untersucht. Dazu wurde eine ELISA-Platte mit rekombinantem, gereinigtem GPIb beschichtet. Bei 77 Hybridomazelllinien wurde eine spezifische Bindung der Antikörper an GPIb festgestellt, 36 Hybride wurden daraufhin kloniert.

In einem zweiten Screening-Schritt wurden die so erhaltenen 36 monoklonalen Antikörper dahingehend untersucht, ob sie, wenn sie an GPIb gebunden sind, die zusätzliche Bindung von VWF an GPIb nicht inhibieren. Dazu wurde ein VWF-Aktivitäts-ELISA gemäß Beispiel 2 durchgeführt. Die Extinktion der Lösung wurde bei 450 nm gemessen. Eine Extinktion > 2500 mE wurde als Kriterium für eine signifikante zusätzliche Bindung von VWF, also für die Eignung der Antikörper verwendet. 17 Antikörper erwiesen sich als geeignet.

Von diesen 17 geeigneten monoklonalen Antikörper wurden die Antikörper 2007-100/05, 2007-98/069 und 2007-101/044 auf Latexpartikel gekoppelt. Alle drei Antikörper eignen sich zur Verwendung als Agonist der Plättchenaggregation, also als Plättchenaktivator, wenn sie an Latexpartikel gekoppelt sind (siehe Beispiel 1).

## Patentansprüche

1. Verfahren zur Bestimmung der GPIb-Rezeptor-abhängigen Plättchenfunktion in einer plättchenhaltigen Probe, wobei die plättchenhaltige Probe mit einem Plättchenaktivator in Kontakt gebracht wird und die Plättchenaktivierung gemessen wird, **dadurch gekennzeichnet, dass** der Plättchenaktivator ein Festphasen-gebundener, GPIb-Bindungspartner ist.

2. Verfahren nach Anspruch 1, wobei der GPIb-Bindungspartner ein GPIb-Antikörper oder ein GPIb-bindendes Antikörperfragment ist.

3. Verfahren nach einem der vorhergehenden Ansprüche, wobei der GPIb-Bindungspartner an eine partikuläre Festphase, bevorzugterweise an Latexpartikel gebunden ist.

4. Verfahren nach einem der vorhergehenden Ansprüche, wobei die plättchenhaltige Probe plättchenreiches Plasma ist.

5. Verfahren nach einem der vorhergehenden Ansprüche, wobei die Plättchenaktivierung gemessen wird, indem die Plättchenaggregation in der Probe gemessen wird.

6. Verfahren zur Diagnose eines Bernard-Soulier-Syndroms,
**dadurch gekennzeichnet, dass** die GPIb-Rezeptor-abhängige Plättchenfunktion in einer plättchenhaltigen Probe eines Individuums mit einem Verfahren gemäß einem der vorhergehenden Ansprüche bestimmt wird und wobei eine verminderte oder fehlende GPIb-Rezeptor-abhängige Plättchenfunktion ein Bernard-Soulier-Syndrom indiziert.

7. Verfahren zur qualitativen oder quantitativen Bestimmung eines GPIb-Inhibitors in einer Probe, **dadurch gekennzeichnet, dass** die GPIb-Rezeptor-abhängige Plättchenfunktion in einer plättchenhaltigen Probe eines Individuums mit einem Verfahren gemäß einem der vorhergehenden Ansprüche bestimmt wird und wobei eine verminderte oder fehlende GPIb-Rezeptor-abhängige Plättchenfunktion die Anwesenheit eines GPIb-Inhibitors indiziert.

8. Verwendung eines GPIb-Bindungspartners, der an eine Festphase gebunden ist, zur Aktivierung von Plättchen in vitro.

9. Verwendung gemäß Anspruch 8, wobei der GPIb-Bindungspartner ein GPIb-Antikörper oder ein GPIb-bindendes Antikörperfragment ist.

10. Verwendung gemäß einem der Ansprüche 8 und 9, wobei der
GPIb-Bindungspartner an eine partikuläre Festphase, vorzugsweise an Latexpartikel, gebunden ist.
